# FASCICULE DE BREVET EUROPEEN

(11) **EP 0 822 184 B1**
(45) Date de publication et mention de la délivrance du brevet: **11.06.2003**
(21) Numéro de dépôt: 97401666.9
(22) Date de dépôt: 10.07.1997
(51) Int. Cl.: C07D 201/02, C07D 207/26, C07D 223/10

(54) **Procédé de préparation de lactames N-substitués**
Verfahren zur Herstellung von N-substituierten Laktamen
Process for the preparation of N-substituted lactams

(30) Priorité: 31.07.1996 FR 9609631
(43) Date de publication de la demande: 04.02.1998
(73) Titulaire: SOCIETE NATIONALE DES POUDRES ET EXPLOSIFS, F-75181 Paris Cédex 04 (FR)
(72) Inventeur: Denarie, Michel, 84210 Pernes les Fontaines (FR); Ly Kok, Khann, 84000 Avignon (FR)

(56) Documents cités:
- EP-A- 0 218 976
- WO-A-96/14299
- US-A- 3 865 814
- US-A- 4 812 566
- US-A- 4 973 688
- HETEROCYCLES, vol. 12, no. 11, 1979, pages 1449-1451, XP000646488

## Description

L'invention a pour objet un procédé amélioré de préparation de lactames N-substitués et plus particulièrement concerne un procédé de préparation de lactames N-substitués, plus économique et plus sûr, par réaction de lactames non substitués sur l'azote avec un halogénure organique.

Les lactames N-substitués peuvent être préparés, selon un procédé habituel, par réaction des sels alcalins de lactames avec des agents d'alkylation, comme décrit par exemple dans le brevet US 4 122 170. Les sels alcalins, utilisés comme composés de départ, doivent être préparés au préalable, par réaction de métaux ou d'hydrures alcalins avec les lactames correspondants, en présence d'un solvant inerte et sous atmosphère d'azote. Ce procédé n'est pas économique en raison du prix élevé des métaux ou hydrures alcalins et des grandes quantités de solvant qui sont nécessaires.

Un autre procédé a été divulgué par G.L. Isele et al (Synthesis, 1971, 266) qui consiste à alkyler le caprolactame avec un bromure ou chlorure d'alkyle en présence de diméthylsulfoxyde comme solvant et d'hydroxyde de potassium comme accepteur d'acide halogénohydrique. Les résultats intéressants de ce procédé seraient dus à l'action du solvant qui favoriserait la formation du sel de potassium du lactame. Mais ce procédé n'est pas économique à l'échelle industrielle. L'hydroxyde de potassium est relativement coûteux. Le solvant, peu habituel, est cher et utilisé en grandes quantités. La séparation du produit obtenu et du diméthylsulfoxyde peut de plus poser des problèmes lorsque les points d'ébullition de ces composés sont proches.

Selon le procédé décrit dans le brevet US n° 3 865 814, on fait réagir les lactames avec un halogénure d'alkyle ou d'aralkyle, en présence d'hydroxyde d'un métal alcalin et en l'absence de solvant, mais néanmoins un solvant est utilisé dans tous les exemples pour récupérer la partie de lactames N-substitués contenue dans le sel formé, séparé par filtration. De plus, en raison de la grande exothermicité de la réaction, il est indiqué qu'il est préférable, pour réaliser la mise en oeuvre du procédé, d'additionner l'hydroxyde alcalin au mélange du lactame et du composé organique halogéné et/ou d'ajouter un solvant inerte avec un point d'ébullition approprié. Des produits secondaires tels que des éthers se forment en grande quantité. Des distillations fractionnées sont alors nécessaires pour récupérer les lactames N-substitués et ces derniers sont encore contaminés par une quantité importante de lactames n'ayant pas réagi. Les rendements sont faibles. Ce procédé n'est par conséquent pas non plus adapté à l'industrie. Les lactames N-substitués ne sont pas suffisamment purs et la chaleur dégagée par la réaction est difficilement contrôlable à l'échelle industrielle.

Plus récemment, sont décrits, dans les brevets US n° 4 812 566 et US n° 4 973 688, des procédés de préparation de 1-dodécylazacycloheptan-2-one, dans lesquels on utilise un catalyseur de transfert de phase et des solvants organiques hydrocarbonés tels que le benzène, le toluène, le xylène, le cyclohexane, le n-hexane et l'éther de pétrole. Des quantités de solvants telles que de 1 à 3 fois le poids total des réactifs doivent être utilisées. Ces solvants sont inflammables et de plus certains sont toxiques comme le benzène et le toluène. Le stockage, le recyclage, l'élimination de ces solvants nécessitent de nombreuses opérations, afin de respecter la sécurité et l'environnement, ce qui augmente notablement le coût du procédé.

L'invention a pour objet un procédé qui ne présente pas les inconvénients des procédés antérieurs, qui est beaucoup plus économique et plus sûr, et qui convient par conséquent parfaitement pour une utilisation industrielle.

Selon le procédé de l'invention, on prépare les lactames N-substitués par réaction d'un lactame non substitué sur l'azote avec un halogénure organique, en présence d'au moins un catalyseur de transfert de phase solide-liquide et d'au moins une base minérale solide, et en l'absence de solvant.

Par l'expression un halogénure organique, on désigne un dérivé halogéné d'hydrocarbure.

L'absence de solvant dans le procédé selon l'invention permet, aussi bien lors de la réaction elle-même que lors des étapes d'isolement des lactames N-substitués, un gain de productivité considérable, une amélioration de la sécurité du procédé et du respect de l'environnement. On évite ainsi les problèmes de stockage et de recyclage des solvants et ceux d'élimination des résidus issus du recyclage. Les lactames N-substitués sont obtenus avec de bons rendements et avec très peu de produits secondaires gênants.

Bien que la réaction selon l'invention génère de l'eau et sans qu'il soit nécessaire de l'éliminer, le procédé est de préférence réalisé en limitant le plus possible la présence d'eau dans le milieu réactionnel. Aussi, on utilise la forme des composés qui contient le moins d'eau, on les sèche éventuellement et on évite l'entrée d'eau dans le réacteur.

Les lactames utilisés comme composés de départ sont les composés représentés par la formule générale : 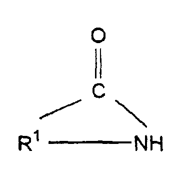 dans laquelle R¹ représente une chaîne hydrocarbonée aliphatique comportant de 2 à 11, de préférence de 3 à 7, atomes de carbone, non substituée ou substituée par un ou plusieurs groupes alkyles linéaires ou ramifiés ayant chacun de 1 à 4 atomes de carbone.

Un seul lactame ou un mélange de lactames peut être utilisé.

Comme exemples de lactames, on peut citer la pyrrolidone, la pipéridone, le caprolactame, le γ-tertiobutylcaprolactame, le mélange des β,β,δ et β,δ,δ triméthylcaprolactames, l'heptanelactame, l'octanelactame, le décanelactame, le dodécanelactame.

Les lactames sont mis à réagir avec des halogénures organiques de formules :

R²-X¹ ou X¹-R²-X²

dans lesquelles X¹ et X², identiques ou différents, représentent un atome d'halogène, tel que de chlore, de brome ou d'iode, de préférence de chlore ou de brome, et R² représente un groupe mono- ou divalent hydrocarboné, saturé ou non, substitué ou non, et de préférence un groupe, substitué ou non, de formules :

R³-CH₂- ou -CH₂-(R^{'3})ₙ-CH₂-

dans lesquelles R³ représente un atome d'hydrogène ou R³ et R^{'3} représentent chacun un groupe hydrocarboné, substitué ou non, monovalent pour R³, divalent pour R^{'3}, ayant de 1 à 17 atomes de carbone, qui peut être formé par des chaînes aliphatiques droites ou ramifiées, ou des noyaux alicycliques ou aromatiques, en une combinaison quelconque et qui peut comporter des liaisons oléfiniques, et n représente le nombre 0 ou 1.

Les substituants des groupes R², R³ et R'³ peuvent être choisis parmi les groupes hydrocarbyloxy ou hydrocarbylthio, tels que des groupes méthoxy, éthyloxy, méthylthio, éthylthio.

Comme exemples d'halogénures organiques, on peut citer : les chlorures, bromures ou iodures de méthyle, d'éthyle, de propyle, de butyle, d'isobutyle, de tertiobutyle, d'allyle, de méthallyle, de pentyle, d'hexyle, d'heptyle, d'octyle, de cyclohexylméthyle, de 3-cyclohexylpropyle, de nonyle, de décyle, d'undécyle, de dodécyle, de tridécyle, de tétradécyle, de pentadécyle, d'hexadécyle, d'heptadécyle, d'octadécyle, de méthoxyéthyle, de benzyle, de 3-(p-méthylphényl)propyle, les dichloruresou dibromures d'éthylène oud'hexylène, le 2-chloro ou 2-bromométhylnaphtalène.

L'halogénure organique et le lactame peuvent être mis à réagir en quantité stoechiométrique. De préférence, on utilise un excès du composé le moins coûteux ou qui s'élimine le plus facilement lors du traitement final, qui est en général le lactame. L'excès est généralement compris entre environ 2 et environ 5% équivalents molaires.

Les bases minérales utilisables dans le procédé de l'invention sont notamment les hydroxydes de métaux alcalins tels que l'hydroxyde de sodium et l'hydroxyde de potassium, les hydroxydes de métaux alcalino-terreux tels que l'hydroxyde de calcium et l'hydroxyde de magnésium. On peut utiliser une seule base ou un mélange de plusieurs de ces bases. Les carbonates de métaux alcalins, tels que le carbonate de potassium, peuvent aussi être employés avec une ou plusieurs de ces bases.

De préférence, pour des raisons économiques, on utilise l'hydroxyde de sodium.

Selon le procédé de l'invention, les bases sont sous forme solide, telle que par exemple sous forme de paillettes, de perles, de poudre ou autre forme similaire, et de préférence sont anhydres.

La quantité de base utilisée peut être comprise entre environ 1,2 et environ 3 équivalents molaires par rapport au réactif en plus faible quantité, et est de préférence d'environ 2 équivalents.

Le schéma réactionnel du procédé avec un halogénure organique monohalogéné est le suivant :

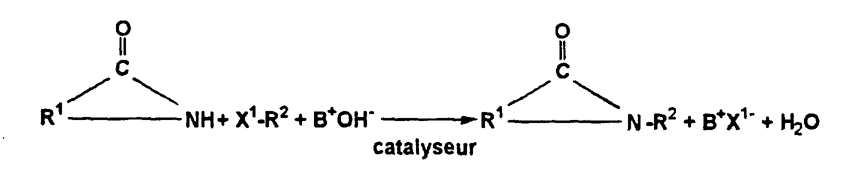

La réaction est effectuée en l'absence de solvant, mais en présence d'au moins un catalyseur de transfert de phase, de type solide-liquide.

Les catalyseurs de transfert de phase qui peuvent être utilisés sont les composés qui sont généralement connus pour être des catalyseurs de transfert de phase solide-liquide, tels que les sels d'onium quaternaire comme les sels d'ammonium, de phosphonium et occasionnellement d'arsonium, les sels de guanidinium, ces sels d'onium ou de guanidinium pouvant être fixés sur un support, les polyéthers comme les polyéthylèneglycols, les éthers-couronnes et les cryptates. De préférence, on utilise une forme anhydre du catalyseur.

Les catalyseurs particulièrement utiles sont les sels d'ammonium quaternaire de formule : 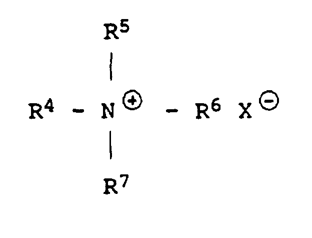 dans laquelle R⁴, R⁵, R⁶ et R⁷ sont des groupes alkyles ou aralkyles ayant de 1 à 18 atomes de carbone, le nombre total d'atomes de carbone contenus dans les groupes R⁴ à R⁷ étant de 10 à 40, de préférence de 16 à 40 ; ou deux ou trois des groupes R⁴, R⁵, R⁶ et R⁷ font partie ensemble et avec l'atome d'azote d'un groupe hétérocyclique, saturé ou non, substitué ou non, le ou les groupes restants étant des groupes alkyles ou aralkyles.

Comme exemples de groupes hétérocycliques, on peut citer les groupes pyridyle, picolyle, pipéridino, morpholino, imidazolyle, pyrrolidinyle, pyrrolyle, pyrrolinyle. X est un anion convenable habituellement utilisé dans ces composés. Comme exemples d'anions, on peut citer les anions halogénures tels que Cl, Br et I, les anions SO₄, SO₄H, OH, perchlorate. De préférence, on utilise les anions Cl, HSO₄ et plus particulièrement Br.

Ces sels d'ammonium peuvent être ou non fixés sur un support, comme il est connu.

La quantité de catalyseur utilisée est généralement comprise entre environ 1 et environ 10% en équivalents molaires, de préférence entre environ 3 et environ 5%, par rapport au réactif en plus faible quantité, généralement par rapport à l'halogénure. Néanmoins une quantité supérieure, si elle n'empêche pas le milieu d'être agité, peut aussi être utilisée.

Les catalyseurs qui conviennent bien sont ceux dont les groupes R⁴, R⁵, R⁶ et R⁷ sont symétriques et qui contiennent chacun au moins 4 atomes de carbone ou ceux dont pas plus de deux de ces groupes ont moins de 4 atomes de carbone. Comme exemple de ces catalyseurs, on peut citer le bromure ou l'hydrogénosulfate de tétrabutylammonium, le chlorure de tricaprylméthylammonium. Le catalyseur préféré est le bromure de tétrabutylammonium.

Le procédé convient particulièrement bien pour l'obtention des lactames N-substitués dont le point de fusion est égal ou inférieur à 60°C.

On peut mélanger tout d'abord le lactame et l'halogénure, chauffer le mélange puis ajouter le catalyseur et ensuite la base de façon telle que la réaction ne s'emballe pas, mais cette mise en oeuvre est cependant difficilement contrôlable et le milieu devient souvent très pâteux ou similaire à un gel. La mise en oeuvre revendiquée du procédé de l'invention consiste à mettre tout d'abord en suspension la base et le catalyseur de transfert de phase, dans l'halogénure organique, puis à ajouter progressivement le lactame, généralement fondu, dans la suspension maintenue à la température de réaction. En suivant cette procédure, le milieu peut, de façon surprenante, être agité facilement, ne se transforme pas en gel et la quantité d'éther produite, impureté non souhaitée, est nettement plus faible.

Dans certain cas, on peut utiliser une pression supérieure à la pression atmosphérique pour faciliter la réaction.

La température de la réaction est généralement comprise entre environ 40° et environ 120°C, de préférence entre 60° et 100°C.

La durée de la réaction dépend de la température, de la quantité de catalyseur et des réactifs utilisés. Elle est généralement comprise entre environ 1 heure et environ 10 heures.

La réaction terminée, les produits contenus dans le mélange réactionnel sont, en raison notamment de l'absence de solvant, facilement séparés par des méthodes classiques.

Selon une mise en oeuvre préférée, lorsque les lactames N-substitués ne sont pas ou sont très peu solubles dans l'eau, on ajoute de l'eau dans le milieu réactionnel pour dissoudre les sels minéraux et organiques hydrosolubles ainsi que les lactames qui n'ont pas réagi. Après décantation et séparation de la phase aqueuse puis, éventuellement, lavage de la phase organique et/ou évaporation de l'eau résiduelle de celle-ci, on obtient déjà les lactames N-substitués bruts avec un bon rendement et une grande pureté.

On peut les purifier par une simple distillation. On les obtient alors avec une pureté généralement supérieure à 99%.

Les lactames N-substitués ont de nombreuses applications. Ils sont utilisés par exemple comme adjuvants des compositions de pesticides, comme solvants ou intermédiaires de matières actives pharmaceutiques ou cosmétiques, comme solvant pour l'extraction de produits de fission nucléaire.

Les exemples qui suivent illustrent l'invention sans toutefois la limiter.

### Exemple 1 : Préparation du N-octylcaprolactame.

Dans un réacteur de 2 litres, à double enveloppe, équipé d'un agitateur mécanique, d'une sonde thermométrique, d'une ampoule de coulée à double enveloppe et d'un réfrigérant, inerté à l'azote, on introduit 526,4 g (3,5 mol) de chlorure d'octyle, 280,3 g (7,0 mol) d'hydroxyde de sodium en perles et 45,8 g (0,14 mol) de bromure de tétrabutylammonium. On chauffe la suspension à 61,5°C, en agitant, et on ajoute lentement, en 4 heures, 408,3 g (3,6 mol) de caprolactame fondu, en maintenant le milieu réactionnel entre 62° et 65°C, puis on continue à agiter le milieu à cette température pendant 5 heures.

On dilue ensuite le milieu réactionnel avec 800 g d'eau, à une température de 65°-70°C. On sépare par décantation la couche organique. On la lave à 65°-70°C, une fois avec une solution aqueuse d'acide sulfurique, puis avec de l'eau jusqu'à un pH constant. Par décantation et après avoir évaporé l'eau résiduelle sous pression réduite, on isole 758,6 g de N-octylcaprolactame brut d'une pureté, déterminée par chromatographie en phase gazeuse (CPG), de 97,8%. Le rendement chimique en N-octylcaprolactame est de 94%.

On distille le N-octylcaprolactame à 138°-143°C/1 mbar et on récupère une fraction de 605 g de N-octylcaprolactame de pureté 99,3% (rendement : 76%).

### Exemples 2 et 3 : Préparation du N-octylcaprolactame.

On prépare le N-octylcaprolactame (NOC) en opérant comme à l'exemple 1, en utilisant la même proportion molaire de lactame, mais en utilisant respectivement le bromure de tétrabutylammonium (TBAB) et l'hydrogénosulfate de tétrabutylammonium (TBAHS) comme catalyseurs. Les conditions et les résultats obtenus sont rassemblés dans le tableau suivant :

| EXEMPLE | 2 | 3 |
|---|---|---|
| CATALYSEUR | TBAB 4% en mol* | TBAHS 4% en mol* |
| NaOH/Chlorure d'octyle mol/mol | 2/1 | 2/1 |
| Température de réaction | 65°C | 65°C |
| Composition du produit brut après 6h de réaction, déterminée par CPG | NOC : 94,6% Chlorure d'octyle : 0,7% | NOC : 91,8% Chlorure d'octyle : 3,15% |

| | | |
|---|---|---|
| * par rapport au chlorure d'octyle. | | |

### Exemples comparatifs 1 et 2 : Préparation du N-octylcaprolactame en présence d'un solvant.

On prépare le N-octylcaprolactame en utilisant, comme solvant, respectivement le toluène et l'oxyde de méthyle et de tertiobutyle (MTBE).

Dans le réacteur, on introduit le solvant, le caprolactame, on ajoute la soude, on chauffe le mélange réactionnel puis on ajoute le bromure de tétrabutylammonium et ensuite progressivement le chlorure d'octyle.

La réaction terminée, on dilue le milieu réactionnel avec de l'eau, on le lave avec une solution aqueuse d'acide sulfurique, puis avec une solution aqueuse de soude et ensuite avec de l'eau et on élimine le solvant par distillation sous pression réduite.

Les conditions réactionnelles et les résultats en comparaison avec ceux obtenus dans l'exemple 1 sont rassemblés dans le tableau 1 suivant.

### Exemples comparatifs 3 à 5 : Préparation de N-octylcaprolactame sans catalyseur et sans solvant.

On utilise le même procédé qu'à l'exemple 1, mais sans utiliser de catalyseur. La température de réaction doit être nettement plus importante pour obtenir une transformation du chlorure d'octyle équivalente. Il en résulte un pourcentage beaucoup plus important des produits secondaires dans le milieu réactionnel.

Les conditions réactionnelles et résultats en comparaison avec ceux obtenus selon l'exemple 1 sont rassemblés dans le tableau 2 suivant.

### Exemple 4 : Préparation du N-dodécylcaprolactame avec du bromure de dodécyle.

Dans un réacteur de 0,5 litre, équipé et traité comme dans l'exemple 1, on introduit 124,6 g (0,50 mol) de

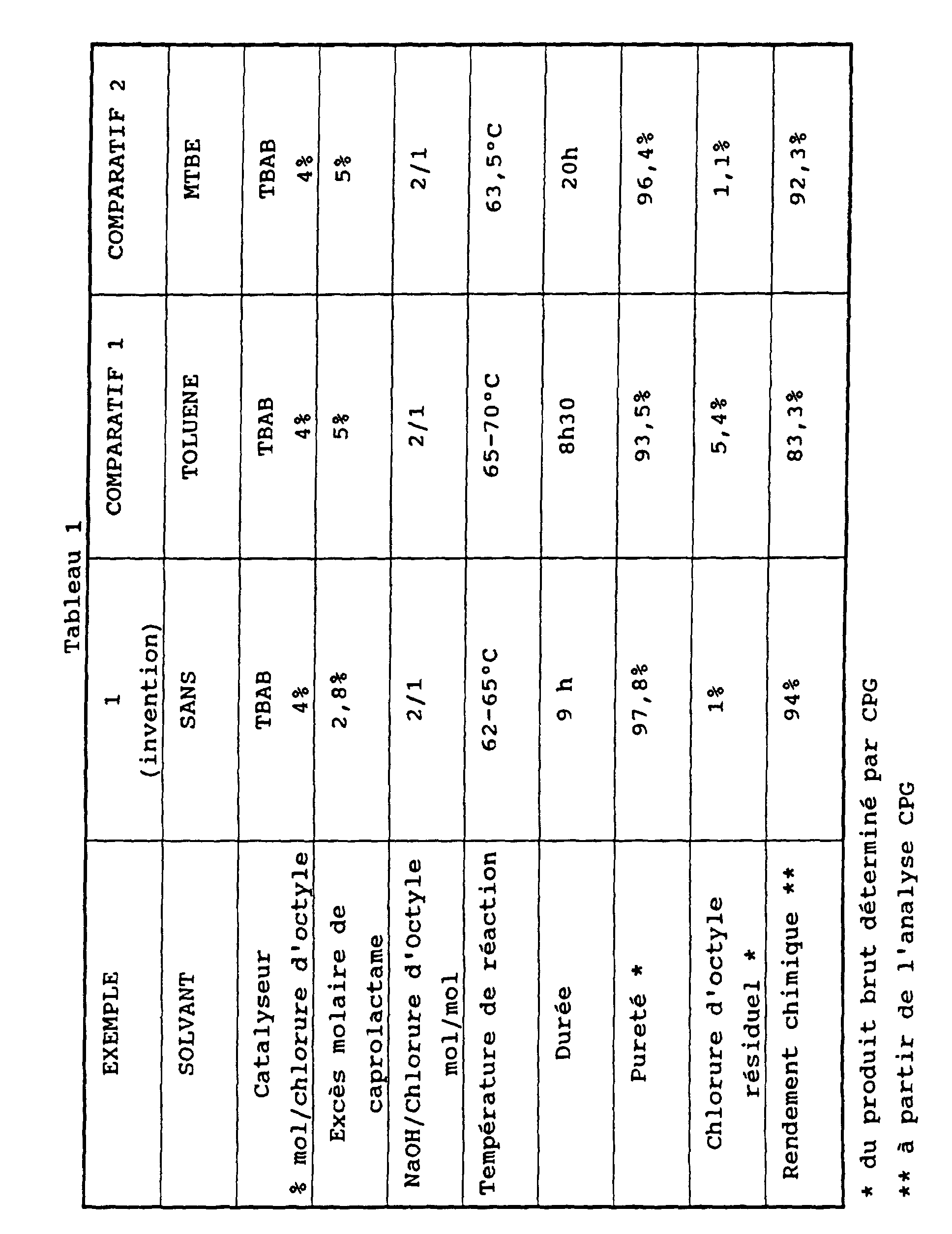

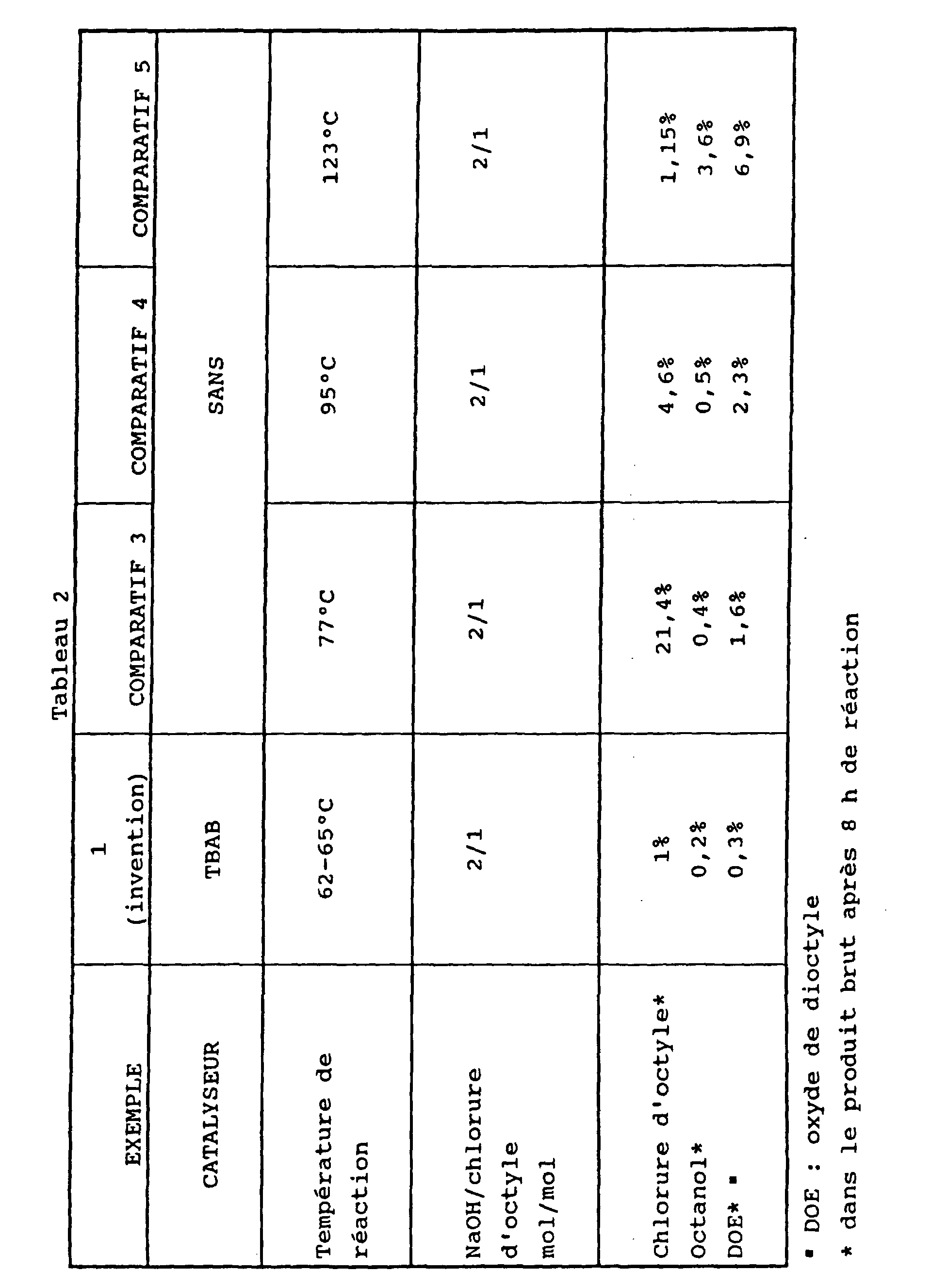

bromure de dodécyle, 40 g (1,0 mol) d'hydroxyde de sodium en perles et 6,44 g (0,02 mol) de bromure de tétrabutylammonium. On chauffe la suspension entre 60° et 65°C, en agitant, et on ajoute lentement, en 1 heure 15 min, 57,7 g (0,51 mol) de caprolactame fondu, en maintenant le milieu réactionnel entre 64° et 67°C, puis on continue à agiter le milieu à 65°C, pendant 7 heures 30 min.

On dilue ensuite le milieu réactionnel avec 110 g d'eau, à 65°C. On sépare par décantation la couche organique. On la lave aux environs de 65°C, une fois avec une solution aqueuse d'acide sulfurique, puis avec de l'eau jusqu'à un pH constant. Par décantation, on isole 136,5 g d'un produit brut dont la composition (déterminée par CPG) est la suivante :

| | |
|---|---|
| N-dodécylcaprolactame | 88,5% |
| dodécanol | 2,1% |
| oxyde de di-dodécyle | 2,9% |
| H₂O | 3,9% |

Le rendement chimique en N-dodécylcaprolactame est de 86%.

On effectue une distillation à 160°-165°C/0,7-0,5 mbar. On récupère ainsi 117,2 g de N-dodécylcaprolactame de pureté 96% (rendement : 80%).

### Exemple 5 : Préparation du N-dodécylcaprolactame avec du chlorure de dodécyle.

Dans un réacteur de 0,5 litre, équipé et traité comme dans l'exemple 1, on introduit 102,4 g (0,50 mol) de chlorure de dodécyle, 40 g (1,0 mol) d'hydroxyde de sodium en perles et 6,44 g (0,02 mol) de bromure de tétrabutylammonium. On chauffe la suspension entre 60° et 65°C, en agitant, et on ajoute lentement, en 1 heure, 57,7 g (0,51 mol) de caprolactame fondu, en maintenant le milieu réactionnel entre 60° et 65°C, puis on continue à agiter le milieu à cette température, pendant 8 heures.

On dilue ensuite le milieu réactionnel avec 110 g d'eau, à 65°C. On sépare par décantation la couche organique. On la lave aux environs de 65°C, une fois avec une solution aqueuse d'acide sulfurique, puis avec de l'eau jusqu'à un pH constant. Par décantation, on isole 138,5 g d'un produit brut dont la composition (déterminée par CPG) est la suivante :

| | |
|---|---|
| N-dodécylcaprolactame | 88% |
| dodécanol | 1,1% |
| oxyde de di-dodécyle | 2,6% |
| H₂O | 3% |

Le rendement chimique en N-dodécylcaprolactame est de 87%.

### Exemple comparatif 6 : Préparation du N-dodécylcaprolactame en présence d'oxyde de méthyle et de tertiobutyle comme solvant.

Dans un réacteur de 250 ml, équipé et traité comme dans l'exemple 1, on introduit 81 g d'oxyde de méthyle et de tertiobutyle, 17,8 g (0,157 mol) de caprolactame, 18,9 g (0,47 mol) d'hydroxyde de sodium en perles, 2,03 g (0,006 mol) de bromure de tétrabutylammonium et 37,4 g (0,15 mol) de bromure de dodécyle.

Le mélange est agité et chauffé à 60°C pendant 9 heures 30 min. On le traite ensuite comme dans les exemples comparatifs 2 et 3.

On obtient 34,5 g de N-dodécylcaprolactame brut de pureté 86%.

Le rendement chimique en N-dodécylcaprolactame est de 72%.

### Exemple 6 : Préparation de la N-octylpyrrolidone en présence de bromure de tétrabutylammonium.

Dans un réacteur de 2 litres, équipé et traité comme dans l'exemple 1, on introduit 538,2 g (3,6 mol) de chlorure d'octyle, 289,6 g (7,2 mol) d'hydroxyde de sodium en perles et 46,5 g (0,14 mol) de bromure de tétrabutylammonium. On chauffe la suspension à 80°C en agitant et on ajoute, lentement, en 2 heures 30 min, 314,3 g (3,7 mol) de pyrrolidone fondue, en maintenant le milieu réactionnel entre 80° et 100°C, puis on continue à agiter le milieu à 80°C, pendant 6 heures.

On dilue ensuite le milieu réactionnel à 60°C avec 628,5 g d'eau. On sépare par décantation la couche organique. On la lave à 50°-60°C, une fois avec une solution aqueuse d'acide sulfurique, puis avec de l'eau jusqu'à un pH constant. Par décantation, on isole 772,5 g d'un produit brut dont la composition est la suivante :

| | |
|---|---|
| N-octylpyrrolidone | 76% |
| octanol | 2,3% |
| oxyde de di-octyle | 4,1% |
| H₂O | 12,2% |

Le rendement chimique en N-octylpyrrolidone est de 83%.

On distille le produit brut à 122°-127°C/2-3 mbar, on récupère ainsi une fraction de 447 g de N-octylpyrrolidone de pureté > 99% contenant moins de 1% d'oxyde de dioctyle (rendement : 63%).

### Exemple 7 : Préparation de la N-octylpyrrolidone en présence de chlorure de tricaprylméthylammonium.

On opère comme à l'exemple 6, mais on utilise, dans la même proportion molaire, le chlorure de tricaprylméthylammonium, vendu sous la marque ALIQUAT 336, à la place du bromure de tétrabutylammonium.

Le rendement chimique en N-octylpyrrolidone, déterminé à partir de l'analyse en CPG du produit brut, est de 62%.

### Exemple comparatif 7 : Préparation de la N-octylpyrrolidone en présence de l'oxyde de méthyle et de tertiobutyle comme solvant.

Dans un réacteur équipé et traité comme dans les exemples précédents, on introduit 81 g d'oxyde de méthyle et de tertiobutyle, 13 g (0,153 mol) de pyrrolidone, 12 g (0,3 mol) d'hydroxyde de sodium, 1,93 g (0,006 mol) de bromure de tétrabutylammonium et 23,3 g (0,149 mol) de chlorure d'octyle. On chauffe le mélange réactionnel à 80°C pendant 6 heures. On le traite ensuite comme dans les exemples comparatifs 2 et 3.

On obtient 20,8 g de N-octylpyrrolidone brut de pureté 82%.

Le rendement chimique en N-octylpyrrolidone est de 66,7%.

### Exemple comparatif 8 : Préparation de la N-octylpyrrolidone, en l'absence de solvant et de catalyseur.

On opère comme dans l'exemple 6, mais sans ajouter de catalyseur et en chauffant le milieu réactionnel pendant 16 heures à 100°C.

Le rendement chimique en N-octylpyrrolidone, déterminé à partir de l'analyse en CPG du produit brut, est de 12%.

### Exemple 8 : Préparation de la N-dodécylpyrrolidone.

Dans un réacteur de 0,5 litre, équipé et traité comme dans l'exemple 1, on introduit 102,4 g (0,50 mol) de chlorure de dodécyle, 40 g (1,0 mol) d'hydroxyde de sodium en perles et 6,44 g (0,02 mol) de bromure de tétrabutylammonium. On chauffe la suspension à 80°C en agitant et on ajoute, lentement, en 1 heure, 43,41 g (0,51 mol) de pyrrolidone fondue, en maintenant le milieu réactionnel entre 80° et 100°C, puis on continue à agiter le milieu à 80°C, pendant 6 heures.

On dilue ensuite le milieu réactionnel à 60°C avec 88 g d'eau. On sépare par décantation la couche organique. On la lave à 55°C, une fois avec une solution aqueuse d'acide sulfurique, puis avec de l'eau jusqu'à un pH constant. Par décantation, on isole 125,9 g de produit brut dont la composition (déterminée par CPG) est la suivante :

| | |
|---|---|
| N-dodécylpyrrolidone | 81% |
| dodécanol | 1,55% |
| oxyde de di-docécyle | 2,80% |
| H₂O | 9% |

Le rendement chimique en N-dodécylpyrrolidone est de 80%.

### Exemple 9 : Préparation du N-benzylcaprolactame.

On opère comme à l'exemple 1, en utilisant les mêmes proportions de réactifs, mais en remplaçant le chlorure d'octyle par le chlorure de benzyle. La température de réaction est comprise entre 65° et 75°C et la durée totale de la réaction est de 9 heures.

Le rendement chimique en N-benzylcaprolactame est de 85%.

Sa structure est confirmée par couplage CPG/SM (spectrographie de masse).

### Exemple 10 : Préparation de la N-benzylpyrrolidone.

On opère comme à l'exemple 6, en utilisant les mêmes proportions de réactifs, mais en remplaçant le chlorure d'octyle par le chlorure de benzyle. La température de la réaction est comprise entre 75° et 85°C et la durée totale de la réaction est de 8 heures 30 min.

Le rendement chimique en N-benzylpyrrolidone est de 84%.

La structure est confirmée par couplage CPG/SM.

### Exemple 11 : Préparation du N-(3-cyclohexyl)propylpyrrolidone.

On opère comme à l'exemple 6, en utilisant les mêmes proportions de réactifs, mais en remplaçant le chlorure d'octyle par le 1-chloro-3-cyclohexylpropane. La température de la réaction est comprise entre 75° et 85°C et la durée totale de la réaction est de 8 heures 30 min.

Le rendement chimique en N-(3-cyclohexyl)propylpyrrolidone est de 85%.

La structure est confirmée par couplage CPG/SM.

## Revendications

1. Procédé de préparation de lactames N-substitués, **caractérisé en ce qu'**on fait réagir un lactame non substitué sur l'azote répondant à la formule générale 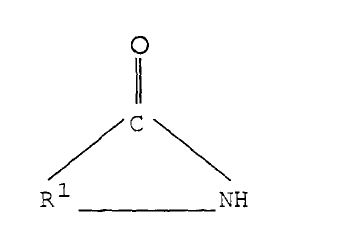 dans laquelle R¹ représente une chaîne hydrocarbonée aliphatique comportant de 2 à 11 atomes de carbone, non substituée ou substituée par un ou plusieurs groupes alkyles linéaires ou ramifiés ayant chacun de 1 à 4 atomes de carbone, avec un dérivé halogéné d'hydrocarbure représenté par les formules générales R³CH₂X¹ ou X¹CH₂(R'³)ₙCH₂X² dans lesquelles X¹ et X², identiques ou différents représentent un atome d'halogène, R³ représente un atome d'hydrogène ou R³ et R^{'3} représentent chacun un groupe hydrocarboné substitué ou non, monovalent pour R³, divalent pour R'³, ayant de 1 à 17 atomes de carbone, qui peut être formé par des chaînes aliphatiques droites ou ramifiées, ou des noyaux alicycliques ou aromatiques, en une combinaison quelconque et qui peut comporter des liaisons oléfiniques, et n représente le nombre 0 ou 1, en mettant en suspension au moins une base minérale solide et au moins un catalyseur de transfert de phase solide-liquide dans le dérivé halogéné d'hydrocarbure, puis en ajoutant progressivement le lactame dans la suspension maintenue à la température de réaction.

2. Procédé selon la revendication 1, **caractérisé en ce que** la chaîne hydrocarbonée de R¹ contient de 3 à 7 atomes de carbone.

3. Procédé selon la revendication 1 ou 2, **caractérisé en ce que** X¹ et X², identiques ou différents, représentent un atome de chlore ou de brome.

4. Procédé selon l'une quelconque des revendications précédentes, **caractérisé en ce que** les substituants de R³ et R^{'3} sont choisis parmi les groupes hydrocarbyloxy ou hydrocarbylthio.

5. Procédé selon l'une quelconque des revendications précédentes, **caractérisé en ce que** la ou les bases sont choisies parmi les hydroxydes de métaux alcalins et les hydroxydes de métaux alcalino-terreux.

6. Procédé selon la revendication 5, **caractérisé en ce que** un ou plusieurs carbonates de métaux alcalins sont utilisés avec la ou les bases.

7. Procédé selon l'une quelconque des revendications précédentes, **caractérisé en ce que** la base est l'hydroxyde de sodium.

8. Procédé selon l'une quelconque des revendications précédentes, **caractérisé en ce que** le catalyseur de transfert de phase est choisi parmi les sels d'ammonium quaternaire de formule : 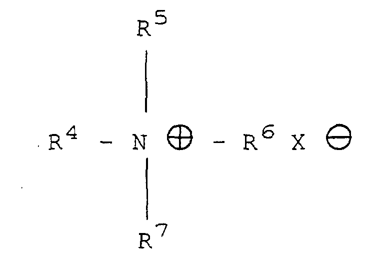 dans laquelle R⁴, R⁵, R⁶ et R⁷ sont des groupes alkyles ou aralkyles ayant de 1 à 18 atomes de carbone, le nombre total d'atomes de carbone contenus dans les groupes R⁴ à R⁷ étant de 10 à 40 ; ou deux ou trois groupes R⁴, R⁵, R⁶ et R⁷ font partie ensemble et avec l'atome d'azote d'un groupe hétérocyclique, saturé ou non, substitué ou non, le ou les groupes restants étant des groupes alkyles ou aralkyles, et X représente un anion.

9. Procédé selon la revendication 8, **caractérisé en ce que** X représente un atome d'halogène ou un hydrogénosulfate.

10. Procédé selon la revendication 8, **caractérisé en ce que** le catalyseur est le bromure de tétrabutylammonium.

11. Procédé selon l'une quelconque des revendications précédentes, **caractérisé en ce que** le procédé est réalisé en limitant la présence d'eau.

12. Procédé selon l'une quelconque des revendications précédentes, **caractérisé en ce que** le lactame et le dérivé halogéné d'hydrocarbure sont mis à réagir en quantité stoechiométrique ou en léger excès de l'un de ces composés, le catalyseur est présent en quantité comprise entre environ 1 et environ 10 % en équivalents molaires par rapport au réactif en plus faible quantité et la base est présente en quantité comprise entre 1,2 et 3 équivalents molaires par rapport à ce réactif.

13. Procédé selon l'une quelconque des revendications précédentes, **caractérisé en ce que** la température de réaction est comprise entre environ 40°C et environ 120°C.

14. Procédé selon l'une quelconque des revendications précédentes, **caractérisé en ce que** la réaction terminée, lorsque le lactame N-substitué n'est pas ou est très peu soluble dans l'eau, on ajoute de l'eau dans le milieu réactionnel et on récupère le lactame N-substitué par décantation.

## Claims

1. Process for preparing N-substituted lactams, **characterized in that** a lactam unsubstituted on the nitrogen, corresponding to the general formula 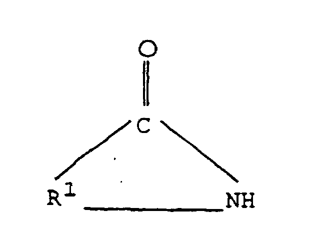 in which R¹ represents an aliphatic hydrocarbon-based chain containing from 2 to 11 carbon atoms, which is unsubstituted or substituted with one or more linear or branched alkyl groups each containing from 1 to 4 carbon atoms, is reacted with a halogenated hydrocarbon derivative represented by the general formulae R³CH₂X¹ or X¹CH₂(R'³)ₙCH₂X² in which X¹ and X², which may be identical or different, represent a halogen atom, R³ represents a hydrogen atom or R³ and R'³ each represent a substituted or unsubstituted hydrocarbon-based group, which is monovalent for R³ and divalent for R'³, containing from 1 to 17 carbon atoms, which may be formed by straight or branched aliphatic chains, or alicyclic or aromatic nuclei, in any combination, and which may comprise olefinic bonds, and n represents the number 0 or 1, by placing at least one solid mineral base and at least one solid-liquid phase-transfer catalyst in suspension in the halogenated hydrocarbon derivative, followed by gradually adding the lactam to the suspension maintained at the reaction temperature.

2. Process according to Claim 1, **characterized in that** the hydrocarbon-based chain of R¹ contains from 3 to 7 carbon atoms.

3. Process according to Claim 1 or 2, **characterized in that** X¹ and X², which may be identical or different, represent a chlorine or bromine atom.

4. Process according to any one of the preceding claims, **characterized in that** the substituents of R³ and R'³ are chosen from hydrocarbyloxy and hydrocarbylthio groups.

5. Process according to any one of the preceding claims, **characterized in that** the base(s) is (are) chosen from alkali metal hydroxides and alkaline-earth metal hydroxides.

6. Process according to Claim 5, **characterized in that** one or more alkali metal carbonates are used with the base(s).

7. Process according to any one of the preceding claims, **characterized in that** the base is sodium hydroxide.

8. Process according to any one of the preceding claims, **characterized in that** the phase-transfer catalyst is chosen from the quaternary ammonium salts of formula: 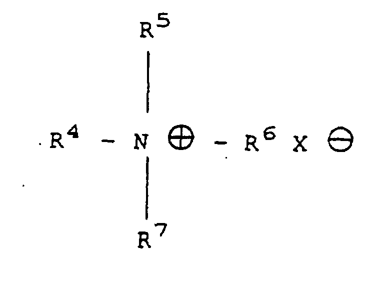 in which R⁴, R⁵, R⁶ and R⁷ are alkyl or aralkyl groups containing from 1 to 18 carbon atoms, the total number of carbon atoms contained in the groups R⁴ to R⁷ being from 10 to 40; or two or three groups R⁴, R⁵, R⁶ and R⁷, together with the nitrogen atom, form part of a saturated or unsaturated, substituted or unsubstituted heterocyclic group, the remaining group(s) being alkyl or aralkyl group(s), and X represents an anion.

9. Process according to Claim 8, **characterized in that** X represents a halogen atom or a hydrogen sulfate.

10. Process according to Claim 8, **characterized in that** the catalyst is tetrabutylammonium bromide.

11. Process according to any one of the preceding claims, **characterized in that** the process is performed while limiting the presence of water.

12. Process according to any one of the preceding claims, **characterized in that** the lactam and the halogenated hydrocarbon derivative are reacted in stoichiometric amount or in a slight excess of one of these compounds, the catalyst is present in an amount of between about 1% and about 10% in molar equivalents relative to the reagent that is in smallest amount, and the base is present in an amount of between 1.2 and 3 molar equivalents relative to this reagent.

13. Process according to any one of the preceding claims, **characterized in that** the reaction temperature is between about 40°C and about 120°C.

14. Process according to any one of the preceding claims, **characterized in that**, once the reaction is complete, when the N-substituted lactam is insoluble or only sparingly soluble in water, water is added to the reaction medium and the N-substituted lactam is recovered by decantation.

## Patentansprüche

1. Verfahren zur Herstellung von N-substituierten Lactamen, **dadurch gekennzeichnet, dass** ein am Stickstoffatom unsubstituiertes Lactam der folgenden allgemeinen Formel: 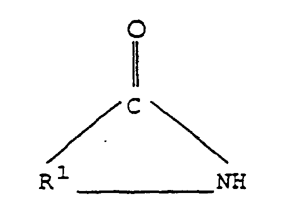 worin die Gruppe R¹ eine aliphatische Kohlenwasserstoffkette mit 2 bis 11 Kohlenstoffatomen bedeutet, die unsubstituiert vorliegt oder mit einer oder mehreren geradkettigen oder verzweigten Alkylgruppen mit jeweils 1 bis 4 Kohlenstoffatomen substituiert sein kann, mit einem halogenierten Kohlenwasserstoffderivat umgesetzt wird, das durch die allgemeinen Formeln R³CH₂X¹ oder X¹CH₂(R'³)ₙCH₂X² dargestellt werden kann, worin die Gruppen X¹ oder X², die gleich oder verschieden sind, ein Halogenatom bedeuten, R³ ein Wasserstoffatom ist oder R³ und R'³ jeweils eine substituierte oder unsubstituierte, im Falle von R³ einwertige und für R'³ zweiwertige Kohlenwasserstoffgruppe mit 1 bis 17 Kohlenstoffatomen bedeuten, die aus geradkettigen oder verzweigten aliphatischen Ketten oder alicyclischen oder aromatischen Ringen in beliebigen Kombinationen bestehen und olefinische Doppelbindungen enthalten kann, und n 0 oder 1 bedeutet, indem mindestens eine feste anorganische Base und mindestens ein Phasentransfer-Katalysator fest-flüssig in dem halogenierten Kohlenwasserstoffderivat suspendiert werden und dann allmählich das Lactam in die auf Reaktionstemperatur gehaltene Suspension gegeben wird.

2. Verfahren nach Anspruch 1, **dadurch gekennzeichnet, dass** die Kohlenwasserstoffkette von R¹ 3 bis 7 Kohlenstoffatome aufweist.

3. Verfahren nach Anspruch 1 oder 2, **dadurch gekennzeichnet, dass** die Gruppen X¹ und X², die gleich oder verschieden sind, ein Chloratom oder ein Bromatom bedeuten.

4. Verfahren nach einem der vorhergehenden Ansprüche, **dadurch gekennzeichnet, dass** die Substituenten der Gruppen R³ und R'³ unter den Gruppen Kohlenwasserstoffoxy oder Kohlenwasserstoffthio ausgewählt sind.

5. Verfahren nach einem der vorhergehenden Ansprüche, **dadurch gekennzeichnet, dass** die Base(n) unter den Alkalimetallhydroxiden und Erdalkalimetallhydroxiden ausgewählt sind.

6. Verfahren nach Anspruch 5, **dadurch gekennzeichnet, dass** als Base(n) ein oder mehrere Alkalimetallcarbonate verwendet werden.

7. Verfahren nach einem der vorhergehenden Ansprüche, **dadurch gekennzeichnet, dass** die Base das Natriumhydroxid ist.

8. Verfahren nach einem der vorhergehenden Ansprüche, **dadurch gekennzeichnet, dass** der Phasentransferkatalysator unter den quartären Ammoniumsalzen der folgenden Formel ausgewählt ist: 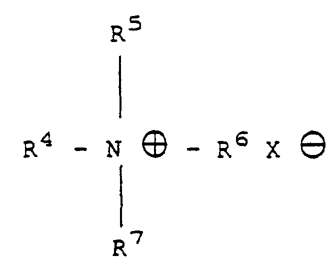 worin die Gruppen R⁴, R⁵, R⁶ und R⁷ Alkyl- oder Aralkylgruppen mit 1 bis 18 Kohlenstoffatomen sind, wobei die Gesamtzahl der Kohlenstoffatome der Gruppen R⁴ bis R⁷ 10 bis 40 beträgt; oder zwei oder drei der Gruppen R⁴, R⁵, R⁶ und R⁷ gemeinsam zusammen mit dem Stickstoffatom eine gesättigte oder ungesättigte, substituierte oder unsubstituierte heterocyclische Gruppe bilden, wobei die verbleibende(n) Gruppe(n) Alkyl- oder Aralkylgruppen sind, und X ein Anion bedeutet.

9. Verfahren nach Anspruch 8, **dadurch gekennzeichnet, dass** X Halogen oder Hydrogensulfat bedeutet.

10. Verfahren nach Anspruch 8, **dadurch gekennzeichnet, dass** der Katalysator das Tetrabutylammoniumbromid ist.

11. Verfahren nach einem der vorhergehenden Ansprüche, **dadurch gekennzeichnet, dass** bei der Durchführung des Verfahrens die Gegenwart von Wasser beschränkt wird.

12. Verfahren nach einem der vorhergehenden Ansprüche, **dadurch gekennzeichnet, dass** das Lactam und das halogenierte Kohlenwasserstoffderivat in einer stöchiometrischen Menge oder bei einem geringen Überschuss einer der Verbindungen umgesetzt werden, der Katalysator in einer in Mol-Äquivalenten ausgedrückten Menge von etwa 1 bis etwa 10%, bezogen auf den in geringerer Menge vorliegenden Reaktanten, vorliegt und die Base in einer Menge im Bereich von 1 bis 3 Mol-Äquivalenten, bezogen auf diesen Reaktanten, vorhanden ist.

13. Verfahren nach einem der vorhergehenden Ansprüche, **dadurch gekennzeichnet, dass** die Reaktionstemperatur im Bereich von etwa 40°C bis etwa 120°C liegt.

14. Verfahren nach einem der vorhergehenden Ansprüche, **dadurch gekennzeichnet, dass** nach abgeschlossener Umsetzung Wasser in das Reaktionsmedium gegeben und das N-substituierte Lactam durch Dekantieren gewonnen wird, wenn das N-substituierte Lactam nicht oder nur sehr wenig wasserlöslich ist.
